# EUROPÄISCHE PATENTSCHRIFT

(11) **EP 0 614 020 B1**
(45) Veröffentlichungstag und Bekanntmachung des Hinweises auf die Patenterteilung: **13.08.1997**
(21) Anmeldenummer: 93810168.0
(22) Anmeldetag: 05.03.1993
(51) Int. Cl.: F16D 3/72, A61B 17/16, B25G 1/02, B23B 31/08

(54) **Vorrichtung zur flexiblen Halterung rotierender Werkzeuge**
Device for flexibly holding rotary tools
Dispositif pour tenir flexiblement des outils rotatifs

(43) Veröffentlichungstag der Anmeldung: 07.09.1994
(60) Teilanmeldung: 96110562.4
(73) Patentinhaber: Sulzer Orthopädie AG, 6340 Baar (CH)
(72) Erfinder: Rothenbühler, Ulrich, CH-3007 Bern (CH)
(74) Vertreter: Hammer, Bruno Dr.

(56) Entgegenhaltungen:
- EP-A- 0 253 526
- EP-A- 0 445 918
- WO-A-88/01026
- CH-A- 290 072
- US-A- 3 068 666
- US-A- 4 706 659
- ANT ANTRIEBSTECHNIK Bd. 18, Nr. 12, 1979, MAINZ DEUTSCHLAND Seiten 628 - 629 SCHUMANN 'Drehsteife Pr zisions-Wellenkupplung Helical'

## Beschreibung

Die Erfindung bezieht sich auf ein Operationsinstrument zur flexiblen Halterung rotierender Werkzeuge gemäss dem Oberbegriff von Anspruch 1.

Operationen am lebenden menschlichen Körper erfordern üblicherweise metallische Hilfsinstrumente. So gelangen zum Beispiel bei der Implantation von Hüftgelenken auch rotierende Werkzeuge wie Bohrer, Fräser oder Schraubenzieher zum Einsatz. Bei gewissen Anwendungen erweist es sich als vorteilhaft, diese Werkzeuge mit einer flexiblen Halterung zu versehen, z.B. dann, wenn Befestigungslöcher zur Befestigung von Hüftgelenkkapseln zu bohren sind. Die Richtung der Bohrung wird dabei üblicherweise mittels einer Bohrlehre vorgegeben, so dass der Operateur durch die entsprechende Führung der Bohrlehre die Richtung des Bohrers bestimmt. Eine flexible Halterung des Bohrers bewirkt, dass der Bohrer der Richtung der Bohrlehre ständig folgt. Aus dem Stand der Technik ist die in Fig. la dargestellte Vorrichtung zur flexiblen Halterung rotierender Werkzeuge bekannt. Die Flexibilität wird dabei durch eine flexible Welle erzielt, die aus mehreren Lagen von schraubenförmig gewickelten Drähten besteht, wobei die Wickelrichtung von einer Lage zur anderen wechselt, um unabhängig vom zwischen Handgriff und Bohrer auftretenden Biegewinkel Torsionskräfte in Drehrichtung zu übertragen. Diese Vorrichtung zur flexiblen Halterung von Werkzeugen weist verschiedene Nachteile auf. Die schraubenförmig gewickelten Drähten reiben während der Rotation aneinander und erzeugen dabei einen metallischen Abrieb, was im Wundbereich zu höchst unerwünschten metallischen Ablagerungen führt. Ein weiterer Nachteil der bekannten Vorrichtung besteht darin, dass eine sterile Reinigung durch die schwer zugänglichen Toträume erschwert ist.

Aus dem Patent US-4,706,659 ist eine weitere Vorrichtung bekannt, welche sich zum Aufbohren des Innenraumes von langen, rohrförmigen Knochen wie Oberschenkelknochen eignet. Das eine flexible Drehmomentübertragungsvorrichtung bildende Teil der Vorrichtung besteht aus einer Mehrzahl von zylinderförmigen Teilabschnitten, welche über Gelenke miteinander verbunden sind. Solche Gelenkstellen weisen unter anderem den Nachteil auf, dass durch die gegenseitige Relativbewegung von zwei Teilabschnitten ein metallischer Abrieb entsteht, welcher sich im Wundbereich ablagert. Weiter ist es äusserst schwierig, die schwer zugänglichen Gelenkflächen steril zu reinigen.

Es ist daher Aufgabe der Erfindung die bekannten Vorrichtungen zur flexiblen Halterung rotierender Werkzeuge derart zu verbessern, dass während der Anwendung kein metallischer Abrieb entsteht und dass die Reinigung der Vorrichtung problemlos ist.

Erfindungsgemäss wird diese Aufgabe gelöst gemäss den kennzeichnenden Merkmalen von Anspruch 1. Die Unteransprüche beziehen sich auf weitere, vorteilhafte Ausführungsformen der erfindungsgemässen Vorrichtung.

Im folgenden wird die Erfindung anhand von Ausführungsbeispielen beschrieben. Es zeigen:
- Fig. 1a: eine bekannte Vorrichtung zur flexiblen Halterung rotierender Werkzeuge;
- Fig. 1b: ein Schnitt (A-A) durch die Halterung gemäss Fig. 1a;
- Fig. 2a: eine flexible Drehmomentübertragungsvorrichtung;
- Fig. 2b: ein Schnitt (B-B) durch die Drehmomentübertragungsvorrichtung gemäss Fig. 2a;
- Fig. 2c: ein Schnitt durch eine weitere Ausführungsform einer Drehmomentübertragungsvorrichtung;
- Fig. 2d: ein Schnitt durch eine weitere Ausführungsform einer Drehmomentübertragungsvorrichtung;
- Fig. 3: eine erfindungsgemässe Vorrichtung zur flexiblen Halterung rotierender Werkzeuge.

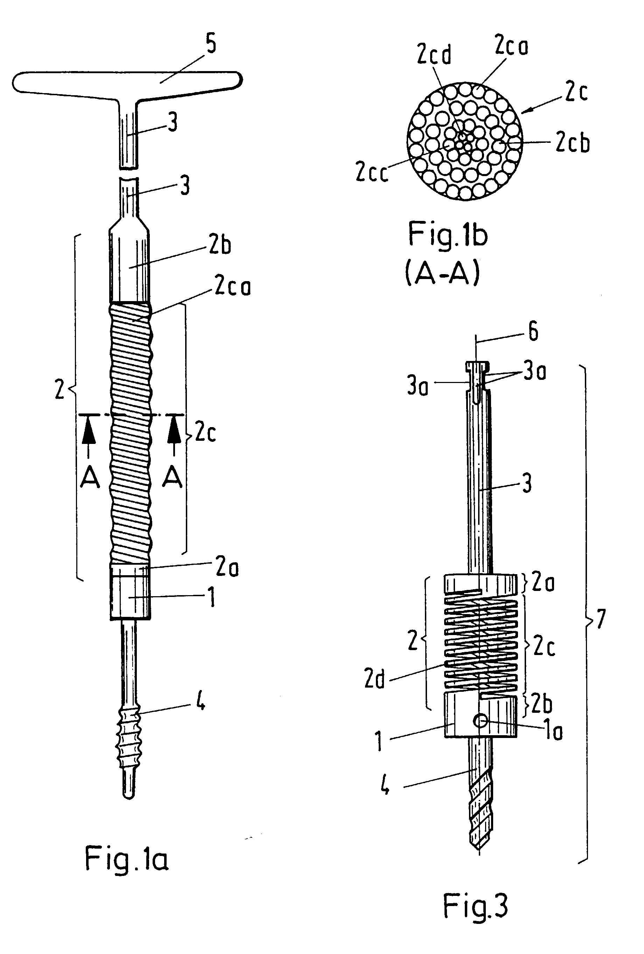

Fig. 1a zeigt eine bekannte Vorrichtung zur flexiblen Halterung von Bohrern, die beim Bohren von Befestigungslöcher zur Verankerung von Hüftgelenkkapseln Verwendung findet. Ein Bohrer 4 ist lösbar in eine Werkzeughalterung 1 eingespannt. Die Werkzeughalterung 1 ist über eine flexible Drehmomentübertragungsvorrichtung 2 mit einer Welle 3, und diese mit einem Handgriff 5 zur Drehmomenteinleitung verbunden. Die flexible Drehmomentübertragungsvorrichtung 2 besteht aus den beiden Endkörpern 2a, 2b und einem dazwischenliegenden, flexiblen Bereich 2c, der aus mehreren Lagen Drahtwicklungen 2ca besteht. Fig. 1b zeigt einen Schnitt (A-A) durch den flexiblen Bereich 2c. Es sind vier unterschiedliche Lagen von Drahtwicklungen 2ca, 2cb, 2cc, 2cd erkennbar, wobei jeweils zwei benachbarte Lagen von Drahtwicklungen in unterschiedlicher Drehrichtung verlaufend schraubenförmig entlang der Längsachse des flexiblen Bereichs 2c gewickelt sind. So ist z.B. die äusserste Drahtwicklung 2ca - von der Werkzeughalterung 1 aus gesehen - entgegengesetzt einer Schraubendrehung gewickelt, wogen die nächstanliegende innere Drahwicklung 2cb in Richtung einer Schraubendrehung gewickelt ist. Die von Lage zu Lage wechselnde Wickelrichtung ermöglicht es, Drehmomente unabhängig von zwischen Handgriff und Bohrer auftretenden Biegewinkeln gleichmässig zu übertragen.

Fig. 3 zeigt eine erfindungsgemässe Vorrichtung 7 zur flexiblen Halterung rotierender Werkzeuge. Die Vorrichtung 7 besteht aus metallischen, in axialer Richtung 6 angeordneten Teilen, einer Werkzeughalterung 1, an der ein Werkzeug 4 lösbar befestigt ist, einer mit der Werkzeughalterung 1 verbundenen, flexiblen Drehmomentübertragungsvorrichtung 2 sowie einer mit der Drehmomentübertragungsvorrichtung 2 verbundenen Welle 3, über die das Drehmoment eingeleitet wird. Die Welle 3, die Drehmomentübertragungsvorrichtung 2 sowie die Werkzeughalterung 4 sind immer einstückig ausgebildet, indem die drei Teile gegenseitig fest und unlösbar angeordnet sind. Die flexible Drehmomentübertragungsvorrichtung 2 weist an deren Enden je einen festen Endkörper 2a, 2b auf und dazwischen einen flexiblen Bereich 2c. Der flexibel Bereich 2c besteht aus einem wendelförmig verlaufenden Streifen 2d, der über mehrere Windungen 2i verlaufend in die entsprechenden Endkörper 2a, 2b ausläuft und mit diesem verbunden ist. Es ist eine Vielzahl unterschiedlicher Vorrichtungen bekannt, um rotierende Werkzeuge in einer Halterung zu fixieren, einzuspannen, einzuklemmen usw. Die dargestellte Werkzeughalterung 1 ist daher nur als ein Beispiel aus einer Vielzahl möglicher Varianten zu betrachten, wobei im vorliegenden Ausführungsbeispiel das rotierende Werkzeug 4 mittels einem Befestigungsmittel 1a, z.B. einer Schraube, an der Werkzeughalterung 1 befestigt ist. Das Drehmoment wird über die Welle 3 eingeleitet, wobei das der Drehmomentübertragungsvorrichtung 2 entgegengesetzte Ende der Welle 3 auf unterschiedlichste Weise ausgestaltbar ist. Dieses Ende kann zum Beispiel einen Handgriff 5 aufweisen, um das Drehmoment von Hand einzuleiten, oder wie in Fig. 3 dargestellt, Ankoppelungsausnehmungen 3a aufweisen, um das Ende der Welle 3 an einen motorischen Antrieb anzukoppeln. Die Vorrichtung 7 zur flexibeln Halterung von Werkzeugen 4 kann aus einem einzigen Werkstück gefertigt sein, oder sich aus mehreren, unterschiedlichen Werkstücken, auch unterschiedlicher Werkstoffe, zusammensetzen, wobei die Werkzeughalterung 1, die Drehmomentübertragungsvorrichtung 2 sowie die Welle 3 gegenseitig fest und unlösbar angeordnet sind. In allen Ausführungsbeispielen ist nur ein wendelförmig verlaufender Streifen 2d dargestellt. Natürlich können die beiden Endkörper 2a, 2b auch durch mehrere wendelförmig verlaufende Streifen 2d verbunden sind. So können z.B. aus dem Endkörper 2a zwei Streifen 2d austreten, indem die Austrittsstellen der beiden Streifen 2d aus dem Endkörper bezüglich der axialen Richtung 6 um 180° verschoben sind. Durch den wendel- oder spiralförmig verlaufenden Streifen 2d erhält der flexible Bereich 2c steife Eigenschaften bezüglich Torsion im axialer Richtung 6 sowie flexible Eigenschaften bezüglich Bewegungen quer zur axialen Richtung 6. Die Länge des flexiblen Bereichs 2c kann durch eine entsprechende Anzahl Windungen des Streifens 2d in axialer Richtung 6 beliebig gestaltet werden. Wird die Werkzeughalterung 1 sehr kurz gestaltet oder als Teil eines Endkörpers 2b ausgebildet, so ergibt sich der Vorteil, dass ein Abbiegen quer zur axialen Richtung 6 unmittelbar hinter einer Werkzeug 4 möglich ist.

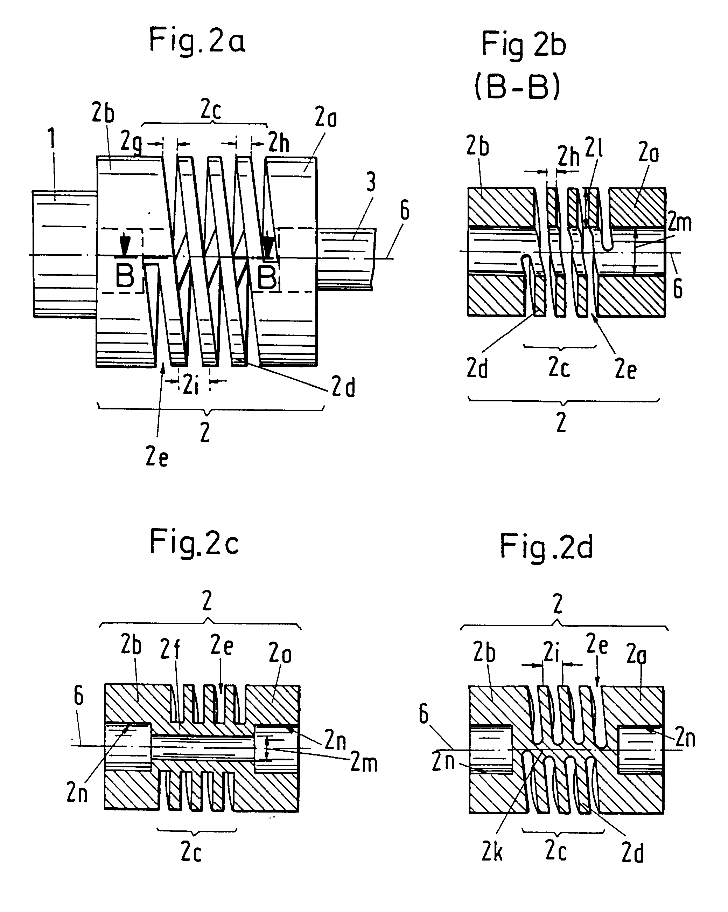

Die Fig. 2a - 2d zeigen unterschiedliche Ausführungsformen flexibler Drehmomentübertragungsvorrichtungen 2. Die flexible Drehmomentübertragungsvorrichtung 2 gemäss Fig. 2a ist aus einem einzigen Werkstück gefertigt. Zwischen den beiden festen Endkörpern 2a, 2b liegt der flexible Bereich 2c. Dieser besteht auf einem wendelförmig um die Achse 6 verlaufenden Streifen 2d, wobei der Streifen 2d eine Streifenbreite 2h und eine radiale Breite 21 aufweist. Zwei benachbarte Windungen weisen einen Abstand 2i auf, wobei der Spalt zwischen den Windungen eine Breite 2g aufweist. Im vorliegenden Ausführungsbeispiel ist die Breite 2g sowie die Streifenbreite 2h über die gesamte Breite des flexiblen Bereichs 2c als konstant dargestellt. Es kann sich jedoch als vorteilhaft erweisen, die Federcharakteristik des flexiblen Bereichs 2c in axialer Richtung 6 zu variieren durch Veränderung der Streifenbreite 2h und/oder des Abstandes 2g. So kann es sich als vorteilhaft erweisen, den flexiblen Bereich 2c im Bereich der Endkörper 2a, 2b steifer zu gestalten als im mittleren Bereich. Am Endkörper 2b ist eine Werkzeughalterung 1 abgebracht, am Endkörper 2a eine Welle 3. Die Endkörper 2a, 2b sind fest an der Welle 3 sowie der Werkzeughalterung 1 angeordnet. Die Werkzeughalterung 1 kann zusammen mit der Drehmomentübertragungsvorrichtung 2 sowie der Welle 3 aus einem einzigen Teil gefertigt sein oder aus mehreren, fest und unlösbar gegenseitig angeordneten Teilen ausgebildet sein, sodass die Werkzeughalterung 1, die Drehmomentübertragungsvorrichtung 2 sowie die Welle 3 immer aus einem Stück bestehen bzw. einstückig ausgebildet sind. Fig. 2b zeigt einen Schnitt (B-B) durch die Fig. 2a. Die Drehmomentübertragungsvorrichtung 2 ist in ihrem Inneren mit einer in axialer Richtung 6 verlaufenden Ausnehmung mit Innendurchmesser 2m versehen, wobei die Innendurchmesser im Bereich der Endkörper 2a, 2b sowie des flexiblen Bereichs 2c auch unterschiedlich sein können. Der Einstich 2e zwischen zwei Windungen des Streifens 2d verläuft von der äusseren Oberfläche des flexiblen Bereichs 2c bis zur inneren Ausnehmung durch, so dass die beiden Endkörper 2a, 2b nur durch ein wendelförmig verlaufenden Streifen 2d verbunden sind. Eine derartige Drehmomentübertragungsvorrichtung 2 weist eine federnde Wirkung in axialer Richtung 6 als auch quer zur axialen Richtung 6 auf.

Fig. 2c zeigt einen Längsschnitt durch ein weiteres Ausführungsbeispiel einer Drehmomentübertragungsvorrichtung 2. Die beiden Endkörper 2a, 2b weisen zylinderförmige Ausnehmungen 2n auf, deren Durchmesser grösser ist, als der Durchmesser der Ausnehmung 2m im flexiblen Bereich 2c. Die beiden Endkörper 2a, 2b sind einerseits durch den wendelförmig verlaufenden Streifen 2d verbunden, wobei der Einstich 2e nicht bis zur inneren Ausnehmung 2m dringt, so dass sich zusätzlich eine zylinderförmige Verbindung 2f ergibt, die die beiden Endkörper 2a, 2b verbindet. Die zylinderförmige Verbindung 2f kann als Vollzylinder ausgeführt sein oder auch Durchbrüche aufweisen, indem gewisse Einstiche 2e bis zum inneren Hohlraum der zylinderförmigen Verbindung 2f durchdringen. Eine derartige Drehmomentübertragungsvorrichtung 2 weist vorwiegend eine federnde Wirkung quer zur axialen Richtung 6 auf.

Fig. 2d zeigt einen Längsschnitt durch ein weiteres Ausführungsbeispiel einer Drehmomentübertragungsvorrichtung 2. Die Endkörper 2a, 2b weisen axiale Ausnehmungen 2n auf. Die beiden Endkörper 2a, 2b sind einerseits durch den wendelförmig verlaufenden Streifen 2d verbunden, wobei der Einstich 2e höchstens bis zur Achse 6 vordringt, derart, dass sich entlang der Achse 6 eine durchgehende, innere Verbindung 2k zwischen den beiden Endkörpern 2a, 2b ergibt. Ein Vorteil der inneren Verbindung 2k besteht darin, dass sie den Abstand 2i zweier benachbarter Windungen vorgibt.

## Patentansprüche

1. Operationsinstrument zur flexiblen Halterung rotierender Werkzeuge (4) für Arbeiten an festen Materialien innerhalb des lebenden, menschlichen Körpers, bestehend aus metallischen, in axialer Richtung (6) aneinandergeordneten Teilen, einer Werkzeughalterung (1) an der ein Werkzeug (4) lösbar befestigt ist, einer mit der Werkzeughalterung (1) verbundenen flexiblen Drehmomentübertragungsvorrichtung (2) sowie einer mit der Drehmomentübertragungsvorrichtung (2) verbundenen Welle (3), über die ein Drehmoment eingeleitet wird, dadurch gekennzeichnet, dass die Werkzeughalterung (4), die Drehmomentübertragungsvorrichtung (2) sowie die Welle (3) einstückig ausgebildet sind, und dass die flexible Drehmomentübertragungsvorrichtung (2) an jedem Ende einen festen Endkörper (2a, 2b) und dazwischen einen flexiblen Bereich (2c) aufweist, dass der flexible Bereich (2c) mindestens aus einem wendelförmigen Streifen (2d) mit mehreren Windungen besteht, der mit den entsprechenden Endkörpern (2a, 2b) verbunden ist, dass die Streifen (2d) einen Querschnitt aufweisen, dessen grössere Dimension radial zur axialen Richtung (6) verläuft, und dass benachbarte Windungen zumindest an deren Peripherie in axialer Richtung einen gegenseitigen Abstand (2g) aufweisen.

2. Operationsinstrument nach Anspruch 1, dadurch gekennzeichnet, dass mindestens zwei wendelförmige Streifen (2d) über mindestens je eine Windung die beiden Endkörper (2a, 2b) verbinden.

3. Operationsinstrument nach einem der Ansprüche 1 oder 2, dadurch gekennzeichnet, dass die wendelförmigen Streifen (2d) auf der inneren, der Achse (6) zugewandten Seite, eine gemeinsame, in axialer Richtung verlaufende innere Verbindung (2k) aufweisen.

4. Operationsinstrument nach einem der Ansprüche 1 oder 2, dadurch gekennzeichnet, dass die wendelförmigen Streifen (2d) auf der inneren, der Achse (6) zugewandten Seite, eine gemeinsame, in axialer Richtung verlaufende zylinderförmige Verbindung (2f) aufweisen.

5. Operationsinstrument nach einem der Ansprüche 1 bis 4, dadurch gekennzeichnet, dass die Welle (3) an dem der flexiblen Drehmomentübertragungsvorrichtung (2) abgewandten Ende einen Handgriff (5) zur manuellen Drehmomenteinleitung aufweist.

6. Operationsinstrument nach einem der Ansprüche 1 bis 4, dadurch gekennzeichnet, dass die Welle (3) an dem der flexiblen Drehmomentübertragungsvorrichtung (2) abgewandten Endbereich derart ausgestaltet ist, z.B. mit Ankoppelungsausnehmungen (3a), dass die Welle (3) an eine motorische Antriebsvorrichtung ankoppelbar ist.

7. Operationsinstrument nach einem der Ansprüche 1 bis 6, dadurch gekennzeichnet, dass die Werkzeughalterung (1) ein lösbares Mittel (1a) zum Einspannen rotierender Werkzeuge (4) aufweist.

8. Operationsinstrument nach einem der Ansprüche 1 bis 7, dadurch gekennzeichnet, dass das Werkzeug (4) ein Bohrer, ein Schraubenzieher oder ein Fräser ist.

## Claims

1. An operating instrument for flexibly holding rotating tools (4) for working on solid materials inside the living human body, consisting of metallic parts arranged one after the another in the axial direction (6), a tool holder (1), to which a tool (4) is detachably attached, a flexible torque transmission device (2) connected to the tool holder (1) and also a shaft (3) connected to the torque transmission device (2), via which a torque is introduced,
**characterised in that** the tool holder (4), the torque transmission device (2) and also the shaft (3) are constructed in one piece,
**and in that** at each end the flexible torque transmission device (2) comprises a fixed end member (2a, 2b) and therebetween a flexible region (2c),
**in that** the flexible region (2c) consists of at least one helical strip (2d) having several windings, which is connected to the corresponding end members (2a, 2b),
**in that** the strips (2d) have a cross section in which the larger dimension extends radially to the axial direction (6),
**and in that** adjacent windings have a mutual spacing (2g) at least on their periphery in the axial direction.

2. An operating instrument according to Claim 1,
**characterised in that** at least two helical strips (2d) connect the two end members (2a. 2b) via at least one winding each.

3. An operating instrument according to one of Claims 1 or 2,
**characterised in that** the helical strips (2d) on the inner side faced towards the axis (6) have a common internal connection (2k) extending in the axial direction.

4. An operating instrument according to one of Claims 1 or 2,
**characterised in that** on the inner side faced towards the axis (6) the helical strips (2d) have a common, cylindrical connection (2f) extending in the axial direction.

5. An operating instrument according to one of Claims 1 to 4,
**characterised in that** at the end faced away from flexible torque transmission device (2) the shaft (3) comprises a handle (5) for the manual introduction of the torque.

6. An operating instrument according to one of Claims 1 to 4,
**characterised in that** at the end region faced away from the flexible torque transmission device (2) the shaft (3) is designed in such a manner, e.g. with coupling recesses (3a), that the shaft (3) can be connected to a motorised driving device.

7. An operating instrument according to one of Claims 1 to 6,
**characterised in that** the tool holder (1) comprises a detachable means (la) for clamping rotating tools (4).

8. An operating instrument according to one of Claims 1 to 7,
**characterised in that** the tool (4) is a drill bit, a screw driver or a milling cutter.

## Revendications

1. Instrument opératoire pour maintenir de façon flexible des outils rotatifs (4) pour travailler sur des matériaux solides à l'intérieur du corps humain vivant, constitué de parties métalliques, disposées les unes contre les autres en direction axiale (6), d'un porte-outil (1) sur lequel un outil (4) est fixé de façon désolidarisable, d'un dispositif de transmission de couple (2) flexible relié au porte-outil (1), ainsi qu'un arbre (3) relié au dispositif de transmission de couple (2) et par l'intermédiaire duquel un couple est introduit, caractérisé en ce que le porte-outil (4), le dispositif de transmission de couple (2) ainsi que l'arbre (3) sont réalisés d'une seule pièce et en ce que le dispositif de transmission de couple flexible (2) présente à chaque extrémité un corps d'extrémité (2a, 2b) rigide et en position intermédiaire une zone (2c) flexible, en ce que la zone flexible (2c) est constituée d'au moins une bande (2d) à forme ondulée ayant plusieurs spires d'enroulement et reliée aux corps d'extrémité (2a, 2b) correspondants, en ce que les bandes (2d) ont une section transversale dont la dimension maximale s'étend radialement par rapport à la direction axiale (6) et en ce que des enroulements voisins présentent au moins sur leur périphérie, en direction axiale, un espacement mutuel (2g).

2. Instrument opératoire selon la revendication 1, caractérisé en ce qu'au moins deux bandes ondulées (2d) relient les deux corps d'extrémité (2a, 2b) chaque fois par au moins un enroulement.

3. Instrument opératoire selon l'une des revendications 1 ou 2, caractérisé en ce que les bandes ondulées (2d) présentent sur la face intérieure tournée vers l'axe (6) une liaison (2k) commune interne s'étendant en direction axiale.

4. Instrument opératoire selon l'une des revendications 1 ou 2, caractérisé en ce que les bandes ondulées (2d) présentent sur la face interne tournée vers l'axe (6) une liaison (2f) commune, cylindrique, s'étendant en direction axiale.

5. Instrument opératoire selon l'une des revendications 1 à 4, caractérisé en ce que l'arbre (3) présente à l'extrémité opposée au dispositif de transmission de couple flexible (2) une poignée (5) destinée à introduire manuellement un couple.

6. Instrument opératoire selon l'une des revendications 1 à 4, caractérisé en ce que l'arbre (3) est équipé sur la zone d'extrémité opposée au dispositif de transmission de couple flexible (2), par exemple d'évidements de couplage (3a), en ce que l'arbre (3) peut être accouplé à un dispositif d'entraînement motorisé.

7. Instrument opératoire selon l'une des revendications 1 à 6, caractérisé en ce que le porte-outil (1) présente un moyen (la) désolidarisable pour serrer des outils rotatifs (4).

8. Instrument opératoire selon l'une des revendications 1 à 7, caractérisé en ce que l'outil (4) est un foret-trépan, un tourne-vis ou bien une fraise.
